# EUROPEAN PATENT SPECIFICATION

(11) **EP 1 630 224 B1**
(45) Date of publication and mention of the grant of the patent: **01.08.2007**
(21) Application number: 05255204.9
(22) Date of filing: 24.08.2005
(51) Int. Cl.: C11D 17/00, C11D 3/37, C11D 3/00

(54) **Thickener for high-PH aqueous systems**
Verdickungsmittel für wässrige Systeme mit hohem pH-Wert
Epaississant pour systèmes aqueux à PH élevé

(30) Priority: 25.08.2004 EP 04292090; 01.03.2005 EP 05290466
(43) Date of publication of application: 01.03.2006
(73) Proprietor: Rohm and Haas Company, Philadelphia, PA 19106-2399 (US)
(72) Inventor: Collin, Jennifer Reichl, Devon, Pennsylvania 19333 (US); Reeve, Paul Francis David, 06935 Grasse (FR); Zeng, Fanwen, Belle Mead, New Jersey 08502 (US)
(74) Representative: Kent, Venetia Katherine

(56) References cited:
- EP-A- 0 329 419
- US-A- 4 836 948
- US-A- 5 529 711
- US-A1- 2004 063 855

## Description

This invention relates to an aqueous system having a high pH and a rheology-modifying polymer.

Rheology modifiers are used in aqueous cleaning products, including for example, shampoo, to increase viscosity at low shear rates while maintaining flow properties of the product at higher shear rates. In addition, rheology modifiers can provide effective, heat-age stable suspensions of particulate material or beads dispersed in the aqueous phase.. A variety of copolymer thickeners made from vinyl monomers have been used for this purpose. For example, U.S. Application Pub. No. 2004/0063855, discloses an acrylic emulsion polymer of methacrylic acid, an alkyl acrylate, acrylic acid and stearyloxypoly(ethyleneoxy)₂₀ethyl methacrylate. However, such thickeners are intended for use in consumer products having near-neutral pH values. In contrast, industrial cleaning compositions and some personal care products can have pH values of at least 10. EP 0329419 discloses cleaning compositions containing crosslinked polymeric thickeners and hypochlorite bleach.

The problem addressed by the present invention is the need for a rheology-modifying polymer having good stability and favorable rheological properties at high pH.

### STATEMENT OF INVENTION

The present invention in its various aspects is as set out in the appended claims.

The present invention provides an aqueous composition having a pH of at least 10, and comprising from 0.1% to 5% of at least one crosslinked copolymer comprising from 2.5% to 65% (meth)acrylic acid residues, from 10% to 80% C₂-C₄ alkyl (meth)acrylate residues, from 2% to 25% lipophilically modified (meth)acrylate residues and residues of a crosslinker that has no ester or amide functionality.

### DETAILED DESCRIPTION

Percentages are weight percentages based on the entire composition, unless specified otherwise. As used herein the term "(meth)acrylic" refers to acrylic or methacrylic, and "(meth)acrylate" refers to acrylate or methacrylate. The term "acrylic polymers" refers to polymers of acrylic monomers, i.e., acrylic acid (AA), methacrylic acid (MAA) and their esters, and copolymers comprising at least 50% of acrylic monomers. Esters of AA and MAA include, but are not limited to, methyl methacrylate (MMA), ethyl methacrylate (EMA), butyl methacrylate (BMA), hydroxyethyl methacrylate (HEMA), methyl acrylate (MA), ethyl acrylate (EA), butyl acrylate (BA), ethylhexyl acrylate (EHA), and hydroxyethyl acrylate (HEA), as well as other alkyl esters of AA or MAA, including the lipophilically modified monomers described below. Preferably, acrylic polymers have at least 75% of monomer residues derived from (meth)acrylic acid or (meth)acrylate monomers, more preferably at least 90%, more preferably at least 95%, and most preferably at least 98%. The term "vinyl monomer" refers to a monomer suitable for addition polymerization and containing a single polymerizable carbon-carbon double bond.

The lipophilically-modified copolymer used according to the invention contains lipophilically-modified (meth)acrylate residues each of which may contain either one, or a plurality of, lipophilic groups. According to one embodiment, such groups are suitably in the same copolymer component as and attached to hydrophilic chains, such as for example polyoxyethylene chains. According to another embodiment, the copolymer may contain a vinyl group which may be used to copolymerize the polymer to other vinyl-containing entities to alter or improve the properties of the polymer. Alternatively other copolymerization systems may be used. The polymerizable group may be attached to the lipophilic group directly, or indirectly for example via one or more, for example up to 60, preferably up to 40, water-soluble linker groups, for example, -CH[R]CH₂O- or -CH[R]CH₂NH- groups wherein R is hydrogen or methyl. Alternatively, the polymerizable group may be attached to the lipophilic group by reaction of the hydrophilic, for example polyoxyethylene, component with a urethane compound containing unsaturation. The molecular weight of the lipophilic-modifying group or groups is preferably selected together with the number of such groups to give the required minimum lipophilic content in the copolymer, and preferably, for satisfactory performance in a wide range of systems.

The amount of lipophilically-modified component in the copolymers useful in the present invention preferably is at least 5%, more preferably at least 10%, and most preferably at least 16%; and preferably is no more than 20%.

The lipophilic-modifying groups themselves are preferably straight chain saturated alkyl groups, but may be aralkyl or alkyl carbocyclic groups such as alkylphenyl groups, having at least 6, and up to 30 carbon atoms although branched chain groups may be contemplated. It is understood that the alkyl groups may be either of synthetic or of natural origin and, in the latter case particularly, may contain a range of chain lengths. For example, naturally sourced stearic acid, even of commercially pure quality may contain only about 90% of stearic chains, up to about 7% of palmitic chains and a proportion of other chains and lower quality products may contain substantially less stearic acid. It is intended herein that reference to the chain length of such groups is to the predominant chain length which is present as more than 50%, preferably in more than 75%, of the chains.

It is an important subsidiary feature of the invention that the chain length of the lipophilic-modifying groups be minimized and the alkyl chain length, or predominant chain length, preferably is below 25, more preferably from 8 to 22, and most preferably from 10 to 18 carbon atoms. The hydrophilic component of the lipophilically-modified copolymer may suitably be a polyoxyethylene component preferably comprising at least one chain of at least 2, preferably at least 5, more preferably at least 10, and up to 60, preferably up to 40, more preferably up to 30 ethylene oxide units. Such components are usually produced in a mixture of chain lengths.

Preferably, the C₂-C₄ alkyl (meth)acrylate residues in the copolymer used in this invention are C₂-C₃ alkyl (meth)acrylate residues, and most preferably EA. Preferably, the amount of C₂-C₄ alkyl (meth)acrylate residues is at least 20%, more preferably at least 30%, more preferably at least 40% and most preferably at least 50%. Preferably, the amount of C₂-C₄ alkyl (meth)acrylate residues is no more than 70%, more preferably no more than 65%, and most preferably no more than 60%. Preferably, the amount of (meth)acrylic acid residues in the copolymer used in the present invention is at least 15%, more preferably at least 17.5%, more preferably at least 20%, and most preferably at least 25%. Preferably, the amount of (meth)acrylic acid residues is no more than 65%, more preferably no more than 50%, more preferably no more than 40%, and most preferably no more than 35%. (Meth)acrylic acid residues are introduced into the copolymer by inclusion of either (meth)acrylic acid, or a (meth)acrylic acid oligomer having a polymerizable vinyl group, in the monomer mixture used to produce the copolymer. Preferably, the copolymer contains residues derived from acrylic acid in an amount no more than 30%, more preferably no more than 27.5%, more preferably no more than 25%, and most preferably no more than 22%. Preferably, the copolymer contains residues derived from acrylic acid in an amount of at least 2.5%, more preferably at least 5%, more preferably at least 7.5%, more preferably at least 10%, and most preferably at least 15%.

Optionally, the copolymer also contains from 2% to 25%, preferably from 5% to 20%, of a hydrophilic comonomer, preferably one having hydroxyl, carboxylic acid or sulfonic acid functionality. Examples of hydrophilic comonomers include 2-hydroxyethyl (meth)acrylate (HEMA or HEA), itaconic acid and acrylamido-2-methylpropanesulfonic acid.

The aqueous compositions of the present invention contain from 0.5% to 5% of at least one copolymer; i.e., the total amount of copolymer(s) is in this range. Preferably, the amount of copolymer in the aqueous composition is at least 0.75%, more preferably at least 1%, and most preferably at least 1.25%. Preferably, the amount of copolymer in the aqueous composition is no more than 4%, more preferably no more than 3%, and most preferably no more than 2.5%. Preferably, the copolymer is an acrylic polymer. The copolymer, in aqueous dispersion or in the dry form, may be blended into an aqueous system to be thickened followed by a suitable addition of acidic or basic material if required.

Preferably, the pH of the aqueous composition is at least 10.5. In one embodiment of the invention, the pH is at least 11. Preferably, the pH is no more than 14, more preferably no more than 13. In one embodiment of the invention, the pH is no more than 12.

The aqueous compositions of the present invention preferably contain from 0.5% to 25% of at least one surfactant; i.e., the total amount of surfactant(s) is in this range. Preferably, the aqueous compositions of the present invention contain at least 1% of at least one surfactant. Preferably, the aqueous composition contains no more than 10%, more preferably no more than 4%, and most preferably no more than 2.5%, of at least one surfactant.

The surfactant(s) preferably is selected from the groups of anionic surfactants characterized by carboxylate, sulfonate, sulfate or phosphate solubilizing groups, and nonionic surfactants characterized by amide or hydroxyl groups or ethylene oxide chains. Cationic, amphoteric or zwitterionic surfactants may also or alternatively be used provided that they are compatible with the thickening copolymer and other ingredients of the aqueous system in the quantity required by the invention. Cationic surfactants characterized by amine or ammonium solubilizing groups, and/or amphoteric surfactants characterized by combinations of the anionic and cationic solubilizing groups may be selected. Preferred surfactants for use in the practice of the invention may be selected from the C₈ to C₁₈ fatty acids or their water soluble salts, water soluble sulfates of C₈ to C₁₈ alcohols, sulfonated alkylaryl compounds such as, for example, dodecylbenzene sulfonate, alkylphenoxy polyethoxy ethanols, for example with C₇ to C₁₈ alkyl groups and 9 to 40 or more oxyethylene units, ethylene oxide derivatives of long chain carboxylic acids, for example of lauric, myristic, palmitic or oleic acids, ethylene oxide derivatives of long chain alcohols, for example of lauryl or cetyl alcohols, alkanolamides and polyglucosides, for example the alkyl polyglucosides. Suitable cationic surfactants may be, for example, lauryl pyridinium chloride, octylbenzyltrimethyl-ammonium chloride, dodecyl trimethylammonium chloride and ethylene oxide condensates of primary fatty acid amines.

The composition of the present invention optionally may include other ingredients, e.g., salts, co-rheology modifiers (e.g. Laponite^{TM} clay, cellulosics, carrageenan, xanthan, other acrylic or urethane rheology modifiers), organic or inorganic particles (including, for example, abrasives, beads, mica, encapsulated oil beads), dispersed liquids, dispersants, biocides, enzymes, bleach, emollient, fragrance, dyes, thioglycolic acid, etc.

The copolymer of the present invention is crosslinked, that is, a crosslinker selected from trimethylolpropane diallyl ether, tetraallyl pentaerythritol, triallyl pentaerythritol, diallyl pentaerythritol and allyl sucroses is included with the copolymer components during polymerization. The crosslinker does not have ester or amide functionality. Trimethylolpropane diallyl ether (TMPDE) and tetraallyl pentaerythritol are especially preferred. The amount of crosslinker residue in the polymer is typically at least 0.01%, preferably at least 0.1%, based on weight of the copolymer components. Preferably, the amount of crosslinker residue in the polymer is no more than 2.5%, more preferably no more than 2.2%. In one embodiment of the invention in which the crosslinker is difunctional, e.g., divinylbenzene, preferably the amount of crosslinker residue in the polymer is at least 0.5%, more preferably at least 1%, and most preferably at least 1.5%. In another embodiment of the invention in which the crosslinking agent is more than difunctional, preferably the amount of crosslinker residue in the polymer is no more than 1.0%, more preferably no more than 0.5%. Additional residues of other crosslinkers which contain ester or amide groups may be present.

In one embodiment of the invention, the copolymer is prepared in the presence of a chain transfer agent when a crosslinking agent is used. Examples of suitable chain transfer agents are carbon tetrachloride, bromoform, bromotrichloromethane, and compounds having a mercapto group, including 3-mercaptopropionic acid or long chain alkyl mercaptans and thioesters such as dodecyl-, octyl-, tetradecyl- or hexadecyl-mercaptans or butyl-, isooctyl- or dodecyl-thioglycolates. When used, the amount of chain transfer agent is typically from 0.01% to 5%, preferably from 0.1% to 1%, based on weight of the copolymer components. If the crosslinking agent is used in conjunction with a chain transfer agent, which are conflicting operations for polymerization purposes, not only is exceptional efficiency observed but also very high compatibility with hydrophilic surfactants, as manifested by increased product clarity.

The rheology modifier may be prepared by copolymerizing the monomers using known aqueous or inverse emulsification procedures at an acidic pH, inverse emulsion polymerization at neutral pH, or precipitation or solution polymerization processes. In such processes, any other suitable additives known in the art, for example, a free-radical initiator such as peroxygen or diazo compounds and, optionally, chain transfer agents may be used. Suitable peroxygen compounds may be peroxides, hydroperoxides, persulfates or organic peroxides and a suitable quantity of initiator may be 0.01% to 3% by weight of the components of the copolymer. The copolymerization temperature is typically 25° C to 92° C, more preferably 60° C to 90° C. The copolymer emulsion may be recovered by filtration and the copolymer may, if desired, be provided in dry form by spray drying or coagulation. U.S. Pat. Nos. 4,384,096, 4,663,385, 4,429,097 and 4,514,552 may be consulted for further general and specific details of suitable copolymerization and recovery techniques, and of suitable monomers and additives. The molecular weight of uncrosslinked lipophilically modified copolymer is typically in the range of about 100,000 to 1 million.

A particular aqueous composition in which the copolymer is useful is a hard surface cleaner. Typical components of a hard surface cleaner, in addition to the copolymer thickener and surfactant mentioned previously, include sufficient base to attain a pH of 10-13, and optional ingredients, including 0.25-5% electrolyte, 1-10% glycol, and 0.5-5% silicate salts, e.g., potassium or sodium silicate or metasilicate. Typically, the surfactant used is either a nonionic or anionic surfactant, or a mixture of both. The copolymer may also be used in abrasive hard surface cleaner, which contains 5-35% calcium carbonate in addition to the ingredients listed above.

Another aqueous composition in which the copolymer is useful is a depilatory. Typical components of a depilatory include the copolymer, thioglycolic acid or its salts, alkali to achieve a pH of 11-13, and optional ingredients, including calcium hydroxide, glycerin, nonionic and/or anionic surfactant, mineral oil, fatty alcohol, and sodium silicate. (See Chemistry and Manufacture of Cosmetics, 3rd Edition, Vol. 2, Ch. 11; Allured Publishing Corp., Carol Stream IL, 2000).

The copolymer also is useful in cream hair relaxer formulations. Other ingredients of these formulations include 1-10% glycol, 5-20% petrolatum, base to achieve pH of 10.5-13, and optional ingredients, including 0.5-10% guanidine, 0.5-10% nonionic surfactant, 1-10% mineral oil, 1-15% fatty alcohol and 0.2-2% cationic polymer. (See Chemistry and Manufacture of Cosmetics, 3rd Edition, Vol. 2, Ch. 17,19; Allured Publishing Corp., Carol Stream Il, 2000).

Other formulations in which the copolymer is useful include hair gel (alcohol-containing and alcohol-free); hair styling cream, paste, or gum; shampoo, conditioner, 2 in 1 conditioning shampoo, body wash/shower gel, liquid soap, sunscreen lotions and sprays, tanning lotions, skin care lotions, two-part hair dyes, permanent waving formulations, textile and hard surface cleaners, e.g., laundry detergent, liquid auto-dish detergent, manual dish detergent, spot-pretreaters, oven cleaners, and glass/window cleaners, and thickening all types of alcohol or water/alcohol formulations. The copolymer may also be used as a polymeric emulsifier with or without co-emulsifiers or surfactants.

### EXAMPLE

### Preparation of Test Samples:

1. Weigh 50g of Hard Surface Cleaner Base¹ into a small beaker.
2. Weigh out sufficient copolymer to have 2.0% copolymer in final formulation. Predilute rheology modifier with 5.0g of deionized water.
3. Add diluted polymer to beaker with stirring. Mix until fully incorporated.
4. Adjust pH of formulation to 11 with 10% NaOH solution.
5. q.s. to 60g total with deionized water. Allow sample to equilibrate for > 1h and adjust pH if necessary with NaOH or citric acid.
6. Split sample. To one portion, add 0.1g of polyethylene beads and mix to incorporate. Second portion will be used for rheology measurements.
7. Measure rheology of sample² after one day at room temperature and after heat aging at 45 °C or 60 °C. Monitor bead suspension at room temperature, 45 °C, 60 °C.
¹Hard Surface Cleaner Base contains 2-3.5% of a blend of anionic and nonionic surfactants and 2-3% of salts, with a pH of approximately 11.
² Rheology analysis conducted on a TA Instruments AR 2000 rheometer at 25 °C with a 60 mm 0.5° steel cone. A standard steady state flow from low to high shear stress method was used for analysis, with shear stress ramp from 0.002 Pa to 700 Pa.

### List of Polymers

| Entry | Copolymer composition |
|---|---|
| Polymer 1 COMPARATIVE | 18 Lipo1^{a}/52EA/10MAA/20AA//0.2DAP /0.1n-DDM |
| Polymer 2 COMPARATIVE | 18 Lipo1/52EA/10MAA/20AA//1.6DVB /0.1n-DDM |
| Polymer 3 COMPARATIVE | 18 Lipo1/52EA/10MAA/20AA//2.0DVB/0.1n-DDM |
| Polymer 4 COMPARATIVE | 18 Lipo1/52EA/10MAA/20AA//1.8 DVB/0.1n-DDM |
| Polymer 5 COMPARATIVE | 18 Lipo1/52EA/10MAA/20AA//0.135 triallyl isocyanurate/0.1n-DDM |
| Polymer 6 COMPARATIVE | 18 Lipo1/52EA/10MAA/20AA//0.15 Methylene-bisacrylamide/0.1n-DDM |
| Polymer 7 | 18 Lipo1/52EA/10MAA/ 20AA//0.12Tetraallyl Pentaerythritol/0.1n-DDM |
| Polymer 8 | 18 Lipo1/52EA/10MAA/ 20AA//0.116Trimethylolpropane Diallyl ether/0.1n-DDM |
| Polymer 9 | 3 Lipo3^{b}/15Lipol/52EA/10MAA/20AA/0.116TMPDE/0.1n-DDM |
| Polymer 10 | 6 Lipo3/12 Lipo1/52EA/10MAA/20AA/0.116TMPDE/0.1n-DDM |
| Polymer 11 | 9 Lipo3/9 Lipo1/52EA/10MAA/20AA/0.116TMPDE/0.1n-DDM |
| Polymer 12 | 18 Lipo1/52EA/10MAA/ 20AA//0.08Tetraallyl Pentaerythritol/0.1n-DDM |

| | |
|---|---|
| a. Lipo1 is a lipophilically modified monomer having a linear saturated C₁₆₋₁₈ alkyl group connected through about 18-26 oxyethylene residues to a methacryloyl group. | |
| b. Lipo3 is a lipophilically modified monomer having a linear saturated C₂₀₋₂₄ alkyl group connected through 20-28 oxyethylene residues to a methacryloyl group. | |
| c. DAP is diallyl phthalate. | |
| d. nDDM is n-dodecyl mercaptan. | |
| e. TMPDE: trimethylolpropane diallyl ether | |

Polymer 1 contains residues of a diallyl phthalate (DAP) crosslinker, which contain ester groups, so that Polymer 1 is not within the scope of the claimed invention and is included for comparative purposes only. Similarly, Polymer 6 contains residues of a methylene-bisacrylamide crosslinker, which contain amide groups, so that Polymer 6 also is not within the scope of the claimed invention and is included for comparative purposes only.

| **2.0% Polymer 2 vs. 2.0% Polymer 1 at pH 11,45 °C** | | | |
|---|---|---|---|
| | | | |
| Sample | Temp / Duration | Viscosity (Pa.s) at Shear Rate of **1e**⁻⁴**/s** | Viscosity (Pa.s) at Shear Rate of **1/s** |
| 2.0% Polymer 2 | Initial | 685 | 5 |
| 2.0% Polymer 2 | 45 °C / 4 weeks | 2077 | 9 |
| 2.0% Polymer 1 | Initial | 1316 | 11 |
| 2.0% Polymer 1 | 45 °C / 4 weeks | 69 | 16 |

| **2.0% Polymer 5 at pH 11, 45°C** | | | |
|---|---|---|---|
| | | | |
| Sample | Temp / Duration | Viscosity (Pa.s) at Shear Rate of **1e⁻⁴/s** | Viscosity (Pa.s) at Shear Rate of **1/s** |
| 2.0% Polymer 5 | Initial | 875 | 15 |
| 2.0% Polymer 5 | 45 °C / 1 week | 552 | 18 |
| 2.0% Polymer 5 | 45 °C / 2 weeks | 579 | 19 |
| 2.0% Polymer 5 | 45 °C / 3 weeks | 507 | 18 |
| 2.0% Polymer 5 | 45 °C / 4 weeks | 374 | 19 |

| **2.0% Polymer 2 vs. 2.0% Polymer 3 vs. Polymer 4 at pH 11, 60 °C** | | | |
|---|---|---|---|
| | | | |
| Sample | Temp / Duration | Viscosity (Pa.s) at Shear Rate of **1e⁻⁴/s** | Viscosity (Pa.s) at Shear Rate of **1/s** |
| 2.0% Polymer 2 | Initial | 642 | 5 |
| 2.0% Polymer 2 | 60 °C / 10 days | 1386 | 10 |
| 2.0% Polymer 3 | Initial | 633 | 5 |
| 2.0% Polymer 3 | 60 °C / 10 days | 3085 | 11 |
| 2.0% Polymer 4 | Initial | 531 | 5 |
| 2.0% Polymer 4 | 60 °C / 10 days | 1278 | 10 |

| **2.0% Polymer 7 at pH 11, 60 °C** | | | |
|---|---|---|---|
| | | | |
| Sample | Temp / Duration | Viscosity (Pa.s) at Shear Rate of **1e**⁻⁴**/s** | Viscosity (Pa.s) at Shear Rate of **1/s** |
| 2.0% Polymer 7 | Initial | 560 | 10 |
| 2.0% Polymer 7 | 60 °C / 7 days | 569 | 12 |
| 2.0% Polymer 7 | 60 °C / 10 days | 435 | 13 |

| **2.0% Polymer 8 at pH 11, 60 °C** | | | |
|---|---|---|---|
| | | | |
| Sample | Temp / Duration | Viscosity (Pa.s) at Shear Rate of **1e⁻⁴/s** | Viscosity (Pa.s) at Shear Rate of **1/s** |
| 2.0% Polymer 8 | Initial | 1199 | 12 |
| 2.0% Polymer 8 | 60 °C / 7 days | 1889 | 17 |
| 2.0% Polymer 8 | 60 °C / 10 days | 1442 | 16 |

| **2.0% Polymer 6 at pH 11, 25 °C vs. 45 °C** | | | |
|---|---|---|---|
| | | | |
| Sample | Temp / Duration | Viscosity (Pa.s) at Shear Rate of **1e⁻⁴/s** | Viscosity (Pa.s) at Shear Rate of **1/s** |
| 2.0% Polymer 6 | Initial | 177 | 14 |
| 2.0% Polymer 6 | 25 °C / 1 week | 208 | 18 |
| 2.0% Polymer 6 | 25 °C / 2 weeks | 175 | 17 |
| 2.0% Polymer 6 | 25 °C / 3 weeks | 155 | 19 |
| 2.0% Polymer 6 | 25 °C / 4 weeks | 150 | 20 |
| 2.0% Polymer 6 | 45 °C / 1 week | 174 | 18 |
| 2.0% Polymer 6 | 45 °C / 2 weeks | 142 | 17 |
| 2.0% Polymer 6 | 45 °C / 3 weeks | 111 | 18 |
| 2.0% Polymer 6 | 45 °C / 4 weeks | 49 | 18 |

The data in the Tables above demonstrate that Polymers 7-8, which are within the scope of the present invention, provide pH-stable high viscosities at low shear rates, and low viscosities at high shear rates. In contrast, Polymer 1, which has ester functionality in its crosslinker residues, and Polymer 6, which has amide functionality in its crosslinker residues, do not provide high viscosity at low shear rate after aging in a high-pH formulation.

## Claims

1. An aqueous composition having a pH of at least 10, and comprising from 0.1 % to 5% of at least one crosslinked copolymer comprising from 2.5% to 65% (meth)acrylic acid residues, from 10% to 80% C2-C4 alkyl (meth)acrylate residues, from 2% to 25% lipophilically modified (meth)acrylate residues and residues of a crosslinker selected from trimethylolpropane diallyl ether, tetraallyl pentaerythritol, triallyl pentaerythritol, diallyl pentaerythritol and allyl sucroses.

2. The composition of claim 1, further comprising from 0.5 to 25% of at least one surfactant.

3. The composition of claim 2 in which the crosslinker is at least one of trimethylolpropane diallyl ether and tetraallyl pentaerythritol.

4. The composition of claim 3 in which the pH is no more than 13.

5. The composition of claim 4 in which said at least one copolymer has from 5% to 25% acrylic acid residues.

6. The composition of claim 5 containing from 0.75 to 4% of said at least one copolymer.

7. The composition of claim 6 in which said at least one copolymer further comprises methacrylic acid residues, and the acrylic acid plus the methacrylic acid residues total from 20% to 40% of the copolymer.

8. The composition of claim 7 in which the residues of a crosslinker that has no ester or amide functionality are present in the copolymer in an amount from 0.1 % to 2.5%.

9. The composition of claim 8 in which said at least one copolymer contains from 16% to 20% lipophilically modified (meth)acrylate residues.

10. The composition of claim 9 in which said at least one surfactant is present in an amount from 1 % to 5%.

## Patentansprüche

1. Wäßrige Zusammensetzung mit einem pH von mindestens 10 und umfassend von 0,1% bis 5% zumindest eines vernetzten Copolymers, umfassend von 2,5% bis 65% (Meth)acrylsäure-Reste, von 10% bis 80% C2-C4-Alkyl(meth)acrylat-Reste, von 2% bis 25% lipophil modifizierte (Meth)acrylat-Reste und Reste eines Vernetzungsmittels, ausgewählt aus Trimethylolpropandiallylether, Tetraallylpentaerythritol, Triallylpentaerythritol, Diallylpentaerythritol und Allylsaccharosen.

2. Zusammensetzung nach Anspruch 1, welche weiter von 0,5 bis 25% zumindest eines grenzflächenaktiven Mittels umfaßt.

3. Zusammensetzung nach Anspruch 2, in welcher das Vernetzungsmittel zumindest eines von Trimethylolpropandiallylether und Tetraallylpentaerythritol ist.

4. Zusammensetzung nach Anspruch 3, in welcher der pH nicht mehr als 13 ist.

5. Zusammensetzung nach Anspruch 4, in welcher das zumindest eine Copolymer von 5% bis 25% Acrylsäure-Reste aufweist.

6. Zusammensetzung nach Anspruch 5, welche von 0,75 bis 4% des zumindest einen Copolymers enthält.

7. Zusammensetzung nach Anspruch 6, in welcher das zumindest eine Copolymer weiter Methacrylsäure-Reste umfaßt, und die Acrylsäure zusammen mit den Methacrylsäure-Resten von 20% bis 40% des Copolymers ausmacht.

8. Zusammensetzung nach Anspruch 7, in welcher die Reste eines Vernetzungsmittels, welches keine Ester- oder Amidfunktionalität aufweist, in dem Copolymer in einer Menge von 0, 1 % bis 2,5% vorliegen.

9. Zusammensetzung nach Anspruch 8, in welcher das zumindest eine Copolymer von 16% bis 20% lipophil modifizierte (Meth)Acrylat-Reste enthält.

10. Zusammensetzung nach Anspruch 9, in welcher das zumindest eine grenzflächenaktive Mittel in einer Menge von 1 % bis 5% vorliegt.

## Revendications

1. Composition aqueuse ayant un pH d'au moins 10, et comprenant de 0,1 % à 5 % d'au moins un copolymère réticulé comprenant de 2,5 % à 65 % de résidus d'acide méthacrylique, de 10 % à 80 % de résidus de méthacrylate d'alkyle en C₂ à C_{4,} de 2 % à 25 % de résidus de méthacrylate modifié de manière lipophile et des résidus d'un agent de réticulation choisi parmi le triméthylolpropane diallyl éther, le tétraallyl pentaérythritol, le triallyl pentaérythritol et les allyl saccharoses.

2. Composition selon la revendication 1, comprenant en outre de 0,5 à 25 % d'au moins un agent tensioactif.

3. Composition selon la revendication 2, dans laquelle l'agent de réticulation est au moins l'un du triméthylolpropane diallyl éther et du tétraallyl pentaérythritol.

4. Composition selon la revendication 3, dans laquelle le pH n'est pas supérieur à 13.

5. Composition selon la revendication 4, dans laquelle ledit au moins un copolymère comprend de 5 % à 25 % de résidus d'acide acrylique.

6. Composition selon la revendication 5, comprenant de 0,75 à 4 % dudit au moins un copolymère.

7. Composition selon la revendication 6, dans laquelle ledit au moins un copolymère comprend en outre des résidus d'acide méthacrylique, et l'acide acrylique plus les résidus d'acide méthacrylique s'élèvent à 20 % à 40 % du copolymère.

8. Composition selon la revendication 7, dans laquelle les résidus d'un agent de réticulation qui n'a ni fonctionnalité ester ni fonctionnalité amide, sont présents dans le copolymère en une quantité de 0,1 % à 2,5 %.

9. Composition selon la revendication 8, dans laquelle ledit au moins un copolymère contient de 16 % à 20 % de résidus de méthacrylate modifié de manière lipophile.

10. Composition selon la revendication la revendication 9, dans laquelle ledit au moins un agent tensioactif est présent en une quantité de 1 % à 5 %.
